# EUROPEAN PATENT APPLICATION

(11) **EP 1 522 309 A1**
(43) Date of publication of application: **13.04.2005**
(21) Application number: 03386018.0
(22) Date of filing: 07.10.2003
(51) Int. Cl.: A61K 35/78, A61P 35/00

(54) **Lipophilic extracts of hypericum perforatum for the therapy of cancer**

(71) Applicant: Skalkos, Dimitrios, 45332 Ioannina (GR); Stavropoulos, Nikolaos E., 45500 Ioannina (GR)
(72) Inventor: Skalkos, Dimitrios, 45332 Ioannina (GR); Stavropoulos, Nikolaos E., 45500 Ioannina (GR)
(74) Representative: Argyriadis, Korinna

(57) **Abstract**

The application describes pharmaceutically active lipophilic extracts from Hypericum perforatum and their use as medicaments to treat cancers, such as bladder cancer. Methods of preparation of the extracts are also provided.

## Description

The invention relates to pharmaceutically active extracts from Hypericum perforatum, and in particular to lipophilic extracts from the plant. These extracts are used as medicaments to treat cancer. Methods of producing the extracts are also provided.

Bladder cancer, related to many environmental carcinogens and tobacco smoke, is a major disease with over 50.000 new cases and approximately 10.000 deaths per year in the U.S.A. alone (1). Variable morphology, natural history, and prognosis demonstrate that transitional cell carcinoma (TCC) of the bladder is not a single disease, but occurs in three distinct forms, each possessing characteristic features (that is, low grade papillary; noninvasive carcinoma in situ; high grade, invasive). Recent studies have begun to elucidate the underlying genetic determinants of the morphologic and biologic characteristics of these different presentations of bladder cancer (2). More than 50% of new bladder cancer cases are superficial or superficially invasive. These types of tumors are considered to have low invasive potential with a progression rate to invasive cancer of 15% to 20%. However, 50% to 90% patients with these types of cancer have recurrence within 12 months after resection (3-5).

The necessity for early adjuvant treatment and mainly intravesical installations of immuno- or chemotherapeutics in the management of high-risk superficial bladder tumors is recognized globally and it is based on the hope that this treatment, by altering the neoplastic potential of the urothelium (6), may hopefully reduce the risk for recurrence and progression, which are the most worrisome problems in the management of superficial bladder cancer (7).

According to a WHO survey, 80% of the world population still rely on herbal medicine as their primary source for therapy (8). In the Western world almost 25% of the active components of currently prescribed drugs were first identified in higher plants (9), and over half of the 50 most used drugs today are derived from plant material (10). Over 60% of chemotherapeutic agents in the treatment of cancer are derived from natural substances (11), and some of the more commonly utilized drugs are still fractionated from plant material (12). The lack of reliable preclinical models, concerns about the consistency and reproducibility of the chemical content, and the absence of knowledge of the mechanisms of actions of botanicals have been cited as obstacles to the pharmacological development of botanical agents.

Hypericum perforatum L (St. John's wort) has been used for centuries in the treatment of burns, bruises, swelling, inflammation, and anxiety, as well as bacterial and viral infections (13). The use of its top flowering parts was originally documented by ancient Greek medical herbalists Hippocrates, Theophrastus, and Dioscorides (14, 15). Locally it is traditionally used both externally and internally with many therapeutic applications (16). The name "hypericum" is derived from Greek and means "over an apparition", a reference to the belief that the herb was so obnoxious to evil spirits, that a whiff of it would cause them to fly. In the last two decades, anti-inflammatory, anti-microbial, antiviral, and antidepressant activities have been attributed to the total extract of the herb or single constituents (17-20). Today hypericum is becoming increasingly popular for the treatment of mild to moderate depression (21, 22). In Germany it is prescribed approximately 20 times more often than fluoxetine one of the most highly prescribed antidepressants in the United States (23). Extract of this drug used in therapy today are prepared by extracting the dried upper aerial part of the plant using ethanol:water or methanol:water mixtures. To date, the antidepressant pharmacological activity of H. perforatum is not fully understood, although from what is known about its mechanism of action the extract may have pharmacological properties similar to conventional antidepressants (22). It is possible to attribute the pharmacological effects not to specific compounds but to the combined action of a variety of its constituents. It is for this reason that the total extract has to be regarded as the active principle of the herb.

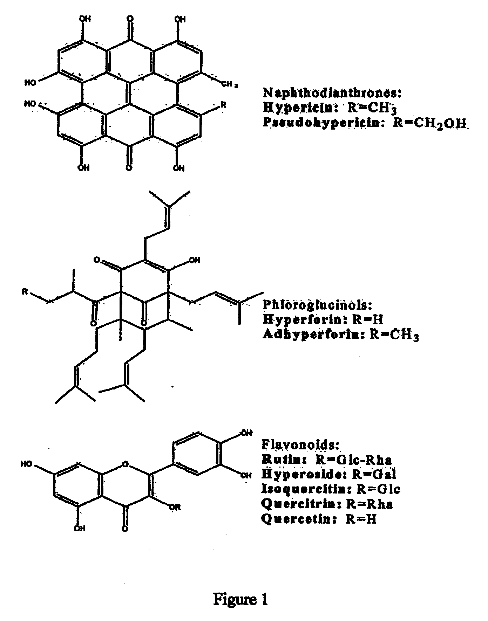

Hypericum perforatum is a plant extract and it contains a complex mixture of phytochemicals. Major constituents of the plant extracts include several classes of compounds exemplified by flavonols, flavonol glycosides, biflavones, naphthodianthrones, phloroglucinols, tannins, coumarins, essential oils and phenylpropanes (24). An indicative analytical composition of the herbal drug is shown in Table 1 (25). The contents of the extracts vary significantly in plants collected from different locations, and depend on the stage of development of the plant. The marketed St. John's wort extracts are standardized in their hypericins (naphthaianthrones) content, as required by the European, and the US pharmacopoeia monographs (26, 27). The anti-depressive activities of the herb were first attributed to hypericin and pseudohypericin, and to flavonoids, but recent pharmacological and clinical results focus on hyperforin as the main active ingredient of the extract (28-30). The structures of these chemicals are shown in Fig 1. All these three groups of compounds are known to be biologically active agents. Hypericin, a photosensitizer with increased interest in recent years, is a photodynamic agent which could be used as a potential clinical photodynamic therapy (PDT) anticancer drug, since several studies have already established its significant in vivo and in vitro antineoplastic activity upon irradiation (31). Quercetin, a flavonoid constituent of the herb widely found in the plant kingdom, inhibits the growth of various malignant cells such as colon carcinoma cell lines, breast cancer cells, cystoadenocarcinoma ovary cell line and others (32-34). Studies on the cytotoxicity of hyperforin, a phloroglucinol constituent, have not been reported yet.

Despite the use by the locals of the herb as a chemopreventive agent for various cancers, there is only one recent report which evaluates the in vitro activity of the ethanolic extract against human malignant cells (35).

The inventors of the present invention have unexpectedly found that a lipophilic extract from the plant still has anticancer activity. This lipophilic extract contains little or no hypericins. Accordingly, a first aspect of the invention provides a lipophilic extract of Hypericum perforatum for use as a medicament to treat cancer.

Also provided, are methods of treating cancer comprising administering a pharmaceutically effective amount of a lipophilic extract of Hypericum perforatum.

By lipophilic extract, we mean a mixture of one or more compounds which may be obtained by extracting an aqueous or alcoholic infusion of Hypericum perforatum with a non-polar solvent which is substantially emissible with the aqueous and/or alcoholic solvent. The non-polar solvent is then removed from the lipophilic extract. Such extracts are termed lipophilic because they are selectively extracted into the non-polar solvent. Preferably, the lipophilic extract contains substantially no detectable hypericins, as detected by the absorption of light at 590 nm, as described herein. This extract may comprise one or more components, such as phloroglucinols, chlorophylls, xanthophylls. The term lipophilic extracts also includes one or more compounds further purified from the lipophilic extract and which have anticancer properties.

By anti-cancer properties, we mean that the extract reduces the proliferation of tumor cells or cancer cell-lines.

Hypericum perforatum is preferably the Greek subspecies known as valsamo or St. John's wort.

Preferably, the lipophilic extract is extracted from the aerial parts of Hypericum perforatum.

Preferably, a method of producing a pharmaceutically active lipophilic extract of Hypericum perforatum is comprising:
(i) extracting at least parts of Hypericum perforatum with a non-polar solvent; and
(ii) removing the non-polar solvent from the lipophilic extract.

Preferably, the invention also provides a method of producing a pharmaceutically active lipopllilic extract of Hypericum perforatum comprising:
(a) Forming an aqueous or alcoholic extract of the flowering parts of Hypericum perforatum in an aqueous and/or alcoholic solvent;
(b) Extracting the extract with a non-polar solvent which is substantially emissible with the aqueous and/or alcoholic solvent;
(c) Separating the non-polar solvent containing the lipophilic extract from the aqueous and/or alcoholic solvent; and
(d) Removing the non-polar solvent from the lipophilic extract.

Preferably, the alcoholic solvent is methanol, although other alcohols such as ethanol may be used. Preferably the aqueous solvent is a buffer, such as phosphate buffered saline pH 7,2. A mixture of an aqueous solvent with an alcohol may also be used.

The aqueous or alcoholic infusion may be prepared by boiling the herb in the aqueous or alcoholic solvent. The solvent may be removed, e.g. by evaporation, and the remaining solid may be redissolved in the same or a different aqueous or alcoholic solvent, prior to extracting with the non-polar solvent.

The non-polar solvent is substantially emissible with the aqueous and/or alcoholic solvent. That is, if the non-polar solvent is mixed with a portion of the aqueous and/or alcoholic solvent, then at least a portion of the non-polar solvent separates into a separate layer to the aqueous and/or alcoholic solvent. This allows the non-polar solvent to be separated from the aqueous and/or alcoholic solvent, for example by pipetting. The non-polar solvent may be removed from the lipophilic extract by, for example, evaporating the extract to dryness under a vacuum.

Preferably, the non-polar solvent is petroleum ether, or hexane, or methylene chloride, or ethyl ether, etc., or mixtures thereof.

The lipophilic extract may be further purified, using techniques known in the art to identify one or more compounds which have anti-cancer activity.

The method also may comprise the additional step of mixing the lipophilic extract with one or more pharmaceutically acceptable carriers, adjuvants or vehicles. Pharmaceutically active compositions obtainable by methods of the invention are also provided.

Pharmaceutically active compositions, according to the invention, for use as medicaments to treat cancer, are also provided, as are methods of treating cancer comprising administering a pharmaceutically effective amount of an extract of the invention.

Most preferably, the cancer treated is a bladder cancer.

Dosages of the extract, when used for the indicated effects, will range between about 10 mg per kg of body weight per day (mg/kg/day) to about 100 mg/kg/day, preferably 20 mg per kg of body weight per day (mg/kg/day) to 50 mg/kg/day, and most preferably 25 to 35. For oral administration, the extract is preferably provided in the form of tablets containing 0,01, 0,05, 0,1, 0,5, 1,0, 2,5, 5,0, 10,0, 15,0, 25,0, 50,0, 100 and 500 mg of the active ingredient for the symptomatic adjustment of the dosage to the patient to be treated. A medicament typically contains from about 0,01 mg to about 300 mg of the active ingredient, preferably from about 1 mg to about 100 mg of active ingredient.

Pharmaceutically acceptable salts of the compounds may be used.

The extract may be used in the form of pharmaceutical compositions.

Pharmaceutical compositions comprising the extracts of the invention, or pharmaceutically acceptable salts thereof, may comprise any pharmaceutically acceptable carrier, adjuvant or vehicle. Pharmaceutically acceptable carriers, adjuvants and vehicles that may be used in the pharmaceutical compositions of this invention include, but are not limited to, ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions may be administered orally, parenterally, by inhalation spray, topically, rectally, nasally, buccally, vaginally, via the bladder or via an implanted reservoir. We prefer oral administration or administration by injection or intravesical installation. The pharmaceutical compositions of this invention may contain any conventional non-toxic pharmaceutically-acceptable carriers, adjuvants or vehicles. The term parenterally as used herein includes subcutaneous, intracutaneous, intravenous, intramuscular, intra-articular, intrasynovial, intrasternal, intrathecal, intralesional, transurethral, intravesical and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a iong-chain alcohol diluent or dispersant such as Ph. Helv or a similar alcohol.

The pharmaceutical compositions may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or colouring agents may be added.

The pharmaceutical compositions may also be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene, polyoxypropylene, emulsifying wax and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention may also be topically applied to the lower intestinal tract by rectal suppository formulation or in a suitable enema formulation. Topically-transdermal patches are also included in this invention.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The invention will now be described by way of example only with reference to the following figures:
Figure 1: Main constituents of hypericum perforatum with biological interest
Figure 2: Extraction procedures for local Hypericum perforatum material
Figure 3: Visible spectra of methanolic extracts HP3, HP4 and HP5
Figure 4: Cytotoxic activity of HP "tea" preparations in the T24 cells. The control are: a) for HP1 , 20 µl of PBS / ml media, and b) for HP2, 5 µl of methanol / ml media. Values of cell densities represent the means of duplicate determinations, which vary by less than 10% of the mean.
Figure 5: Cytotoxic activity of HP extracts in the T24 cells. The control for all experiments is 6 ml of DMSO / ml media. Values of cell densities represent the means of duplicate determinations, which vary by less than 10% of the mean.

### Materials and Methods

### Clinical Study

A small clinical study was conducted with the aim to gather some initial information about the prophylactic value (chemoprevention) of the herb in patients with recurrent superficial bladder tumors in the short term.

### Patients and Methods

A total of 14 patients (12 males and 2 females) with recurrent superficial bladder tumors (Ta/T1, grade 2, 3) initially treated with transurethral resection for their tumors were offered of the herb orally in a daily basis. Recurrence or progression, as terminal events of the study, were recorded. In order to validate the prophylactic value of the treatment, the difference between the post-herbal and the pre-herbal intervals to tumor recurrence was estimated for all patients.

### Dosage

25 gr of the dry herb are boiled in 4,5 Lit. of water by each patient. The aqueous solution is then stored and used for daily oral consumption. One or two classes of the solution (300 ml each) are consumed daily, on a constant basis.

### Results

Tumor recurrence has been detected up to now in 6 patients (42,85%) in a mean follow-up period of 7,5 months. Eight patients (57,14%) remain tumor-free for a mean of 20,5 months, while the mean pre-herbal recurrence-free interval for all patients was 9,8 months. No serious side-effects were noted.

### Conclusions

The daily oral administration of the herb as a "tea" preparation resulted in a slightly longer disease-free interval in a significant proportion of patients. Further studies with larger number of patients and probably higher dose of the herb might be indicated.

### Further Study

### Chemicals

Methanol, and petroleum ether were purchased from Lab-Scan Ltd (Dublin, Ireland). Culture graded Dimethylsulfoxide (DMSO) was obtained from Sigma-Aldrich (St Louis, MO). Dulbecco's modified Eagle's medium (DMEM, 10% fetal cell serum (FCS), penicillin, streptomycin glutamin, PBS were purchased from GIBCO/In Vitrogen. Hypericin was purchased from Sigma-Aldrich ( St. Louis, MO).

### Plant material.

The fresh aerial parts of hypericum perforatum L., known locally as valsalmo were collected from Zagori (Epirus) in Greece, in June 2002. The voucher specimen has been deposited at the Rizarius School's Culture Center K. Lazarides, in Koukouli, loannina, Greece (No. 519). The plant material was dried, pulverized and stored until used (2 kg).

### Extracts Preparation.

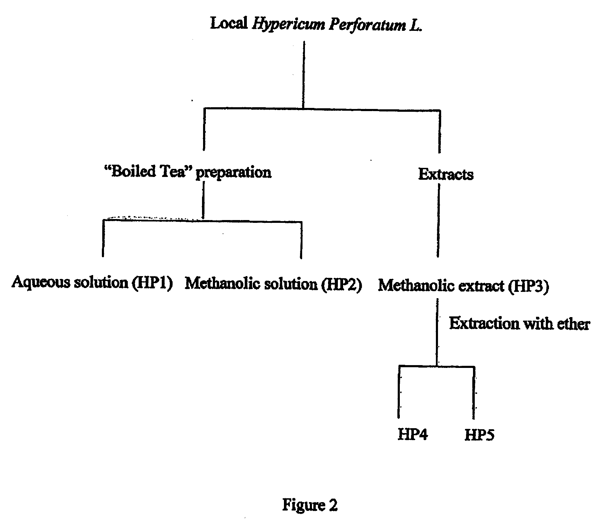

Figure 2 shows the extraction procedures used for the dried part of the plant. Herbal extracts were first prepared as "boiled teas" which is how it is traditionally prepared for per os administration. Aqueous extracted solution was prepared by adding 9 g of dry herb to 100 ml phosphate buffered saline (PBS), bringing this solution to a boil, then letting it boil for 30 min. After the solution had cooled, the extract was decanted away from the residual solids, centrifuged for 10 minutes, and used for in vitro testing the same day (HP1). Methanolic solution was prepared the same way by adding 9 g of dry herb to 100 ml methanol, boiling the solution for 30 min, and using the extract for the in vitro testing the same day (HP2).

Methanolic extract of the herb was prepared by extracting 10 g of dry herb with 100 ml methanol (boiling for 30 min). The solution was concentrated to dryness by evaporation under reduced pressure, to give 1,1 g of extract (HP3). Dry herb 10 g was extracted again with 100 ml methanol by boiling for 30 min. The solution was then extracted three times with equal amounts of petroleum ether. The methanolic fraction was collected, concentrated under vacuum to give 1 g of dry extract (HP4). The ether fraction was collected, evaporated to dryness, under reduced pressure to give 0,1 g of extract (HP5).

### Quantitative determination of hypericins content:

The determination of the total hypericins content in the plant extracts was carried out following the spectrophotometric procedure adapted by U.S. pharmacopoeia (27). The percentage of hypericins was measured in mg of total extract (mg of hypericins in 100 mg of extract). Methanolic solutions were made up for the extracts, and for the standard, hypericin. The visible spectra acquired in the range 450-700 nm were recorded on a UV-Vis. Shimadzu Spectrophotometer, using a cuvette of 1 cm pathlength. Molecular absorption versus hypericins concentration at 590 nm was linear over the range 0 and 2 µg/ml.

### Cell proliferation assay:

T24 bladder cancer cell line was used. This is a cancer cell line derived from a grade 3 human bladder tumor.

T24 cells were cultured in Dulbecco's modified Eagle's medium (DMEM), 1 g/L D-glucose, 10% fetal serum (FCS) supplemented with 100 U/ml penicillin, streptomycin 100 µg/µl, and 2 mM glutamine. After 24hrs, wells received either aliquots of buffer, or buffer containing various concentrations in µl per ml of the HP1 solution in PBS (maximum of 20 µl/ml was used). 48hrs later aliquots of buffer with or without samples were added again. Cells were counted after 6 days with a Coulter Counter. Unless otherwise indicated, values of cell densities represent the means of duplicate determinations, which varied by less than 10% of the mean. Treatment of the cells using 20 µl/ml of PBS as described was used as control in this experiment (control experiment). The same experiment was repeated using HP2 solution in methanol, instead of HP1 in PBS (maximum of 5 µl/ml media was used). In this case treatment of the cells with 5 µl of methanol/ml media was used as the control experiment.

Samples of the extracts HP3, 4 and 5 were dissolved in DMSO prior to the addition to the cells (6,5 mg/ml). Cells received either aliquots of buffer, or buffer containing various concentrations in µl per ml of the HP3, 4, 5 DMSO solutions prepared (maximum of 6 µl/ml). Treatment of cells with 6 µl of DMSO / ml media was used as the control set up for these experiments.

The results of the in vitro experiments were expressed as the percentage reduction of cell colonies when compared to the controls in each case.

### Results

Solutions of the herb with water (HP1), or methanol (HP2) were prepared by boiling 9 g of dry material in 100 ml of solvent for 30 minutes. The color of the solution was red caused by the hypericins contained in the herb. Extraction of the herb with methanol provided 11% extracted material (from 10 g of dry herb, 1,1 g were extracted as HP3). Fractionation of this extract with petroleum ether resulted in the two fractions: HP4, 90% (1 g from 1,1 g of extract), and HP5, 10% (0,1 g from 1,1 g of extract).

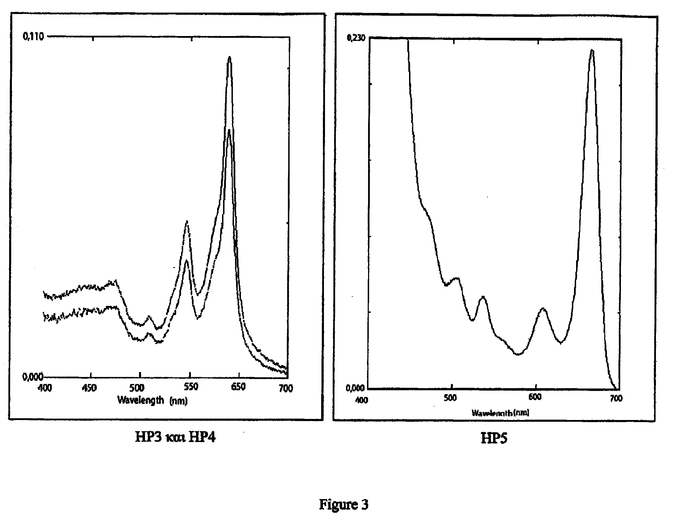

Figure 3 shows the visible spectra of the methanolic extracts of the herb recorded. The spectrum of the total methanolic extract HP3 is similar to the spectrum of the fraction HP4 which contains the polar constituents of the extract. Both exhibit a strong absorption at 590 nm which is characteristic of the hypericins' components of the herb such as hypericin, pseudohypericin, protohypericin and protopseudohypericin (36). Quantitative determination of hypericins in these two extracts, comparing the λ 590 nm absorptions with the absorption of the standard hypericin solution, indicated that extract HP3 contained 1,32% hypericins, and HP4 contained 1,37% (mg of hypericins per 100 mg of extract). The spectrum of the fraction HP5, which contains the non polar constituents of the methanolic extract, is shown in Figure 3, and it exhibits a strong absorption at 660 nm which is characteristic of the chlorophyll(s) found in the plants (37). In this extract there was no detectable absorption band at 590 nm in order to determine hypericins concentration.

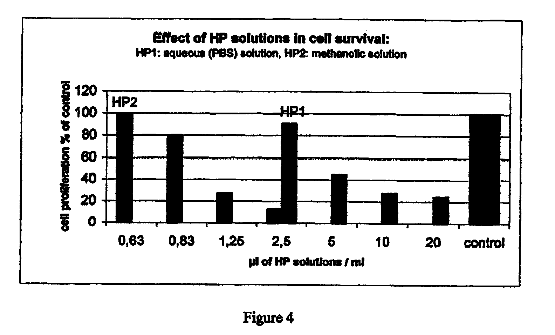

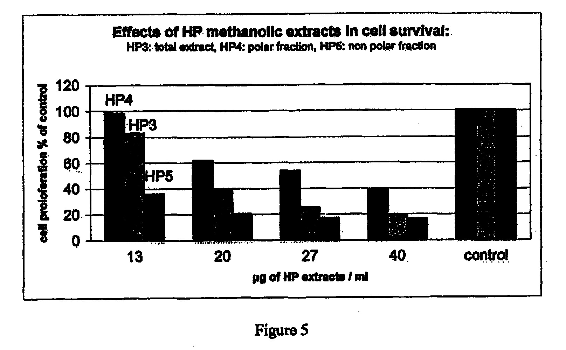

The cytotoxicity of the solutions and the extracts of the local hypericum perforatum was determined in the T24 bladder tumor cell line. Figure 4 shows the antiproliferative activity of the aqueous (HP1), and methanolic (HP2) solutions. The methanolic solution was more effective than the aqueous solution (LC₅₀: 1,1 µl/ml and 4,7 µl/ml respectively), and it inhibited cell growth by 90%, at 2,5 µl/ml. Figure 5 shows the effect on cell growth of the methanolic extract, and its fractions. The total extract HP3 induced an 80% cell growth inhibition at a low concentration of 40 µg/ml, with LC₅₀ value of 18 µg/ml. Fractionation of the methanolic extract provided a less active fraction (HP4), and a more active fraction (HP5). The fraction with the polar constituents-HP4 inhibited cell growth by only 40% at 40 µg/ml (LC₅₀ value of 29 µg/ml), where the fraction with the non polar constituents-HP5 inhibited cell growth by 85% at the same concentration of 40 µg/ml (LC₅₀ value of 10 µg/ml). The most effective extract proved to be the HP5 which exhibited significant cell inhibition at very low concentrations: 83% (at 27 µg/ml), 80% (at 20 µg/ml), and 65% (at 13 µg/ml). All three extracts HP3, 4, and 5 showed very similar dose-effect responses as shown in Figure 5.

### Discussion

One of the main problems in urology in the management of superficial bladder cancer, is the selection of the optimal treatment strategy in terms of both the type of agent and its dosage scheme that should be administered. Although the superiority of any of the commonly used intravesical drugs has never been demonstrated (38, 39), most authors suggest that immunotherapy with bacillus Calmette-Guerin (BCG) from Mycobacterium bovis is more effective to commonly used chemotherapeuticals in terms of reduction of tumor recurrence, treatment of residual papillary transitional cell carcinoma, and treatment of carcinoma in situ (40, 41). Unfortunately, BCG immunotherapy is not devoid of frequently seen mild to severe side effects (42). Moreover, despite the intensive research on this area, 50-60% of superficial bladder tumors continue to recur, and as many as 30% of these recurrent tumors present with a higher grade and/or progress to muscle invasive disease (7). For all the above reasons the exploration of new treatment strategies and new medicines for the prevention of the recurrence and progression of high-risk superficial bladder cancer seems warranted. The latter is reflected in recent and older bibliography with various studies of new substances tested against bladder cancer cell lines.

Among them bropirimine has shown that it may be useful for the prophylaxis of recurrence following transurethral resection of superficial bladder cancer and has a good safety profile and oral activity (43). The significant anti-tumor efficacy of subcutaneous and oral administration of allium sativum (AS) (garlic) warrants further investigation and suggests that AS may provide a new and effective form of therapy for transitional cell carcinoma of the bladder (44). Retinoids modulate the growth and differentiation of normal and malignant epithelial cells in vitro and in vivo, and inhibit bladder carcinogenesis in animal models (44). The anti-tumor effects of Scutellariae radix and its components baicalein, baicalin, and wogonin on human bladder cancer cell lines (KU-1 and EJ-1) and a murine bladder cancer cell line (MBT) were studied recently (45). The results suggest that these traditional Chinese herbal medicines may become an attractive and promising treatment for bladder cancer. The effects of other natural substances like isoflavones (like genistein, daidzein, and biochanin-A) contained in soybean food have been studied in a total of seven human bladder cancer cell lines and an immortalized uroepithelial cell line either individually or as an equal-proportion mixture regimen, on cell growth, DNA synthesis, alterations of cell cycle distribution, and induction of apoptosis. The results seem to justify the potential use of soybean foods as a practical chemoprevention approach for patients with urinary tract cancer (46).

A useful strategy for the discovery of new biologically active compounds from plants is the "ethnopharmacological approach", which utilizes information about the traditional medicinal use of plants for selected diseases (47). An advantage of this strategy over random screening is that extensive clinical tradition and literature may allow for some rationalization with respect to the biological potential of a reputed use. The present study was undertaken to evaluate the growth inhibitory activity of the local hypericum perforatum L. against bladder cancer, and identify the chemical components which may be interested for this activity. The antitumor activities of the particular herb were reported for the first time recently (35). Ethanolic total extracts of the herb were tested against glioblastoma cell lines and normal human astrocytes.

The viability of all cell lines tested were affected comparably under the test conditions determining an LC₅₀ value between 1,9-4,1 mg/ml, after 48hr treatment with extracts from dry and fresh plants. In our study the LC₅₀ value was found between 10-29 µg/ml, after 6days from the treatment of T24 bladder cells with methanolic dry extract. The significant difference in the cytotoxicity between the two herbs can be attributed: a) to the different cell lines used, b) to the different composition of the plants used, c) to the different experimental set up adopted in the two studies. The T24 cell line however represents the most malignant cell line of this type of cancer.

Comparison of the above mentioned natural substances tested against bladder cancer, with the Hypericum perforatum extracts of our study can be made in selected cases, and it is worth noticing. Allium sativum's anti-tumor activity against MBT2 murine tumor cells in vitro required a very high concentrations of 250 mg/ml for a 98,5 % cell death, compared to a concentration of 40 µg/ml for a cell killing of 85 % of the Hypericum perforatum fraction (HP5) in our study. The herb Scutellariae radix at a concentration of 56 µg/ml exhibited a 50% inhibition of the highly malignant KU-1 bladder cancer cell line, compared to an equally effective concentration of 18 µg/ml and 10 µg/ml of the total extract (HP3), and of the non polar fraction of Hypericum perforatum (HP5) in this study.

In this work for the first time we have tried to identify the chemical components of the herb which may be responsible for the antiproliferative activity of the herb. These chemicals may act alone or synergistically with other components. Initially we compared the antiproliferative effect of the aqueous and methanolic solutions (HP1, and HP2) and found the methanolic solution was more active against the T24 tumor cells used (Fig. 4). This result prompted us to continue with extraction of the herb with methanol, rather than water. The total extract produced HP3 was fractionated in two part using methanol, and petroleum ether as solvents (HP4 and HP5 respectively). The fraction HP4 represents 90% of the extract, whereas HP5 represents 10% of the extract. The activity order on the particular cancer cell line of these extracts, tested under the same conditions, was found to be HP5>HP3>HP4 (Fig. 5). The most active fraction is the one which contains primarily the lipophilic compounds of the herb's extract (LC₅₀ values for the three fractions 10 µg/ml - for HP5 > 18 µg/ml - for HP3 > 29 µg/ml - for HP4. According to a recent report this fraction contains among others phloroglucinols, xanthophylls, chlorophylls and unidentified constituents (48). The presence of chlorophyll in this fraction is indicated by the characteristic absorption band at 660 nm shown in Figure 3. Chlorophylls are reported to have chemopreventive properties in rainbow trout (49). Hyperforin, the main phloroglucinol, is considered to be the chemical primarily significant for the anti-depressive activity of the herb (50). The less active fraction contains the majority of the herb's chemicals, which are hydrophilic. Some of them are shown in Table 1. The contribution of these constituents in the overall anti-tumor activity of the herb shouldn't be excluded, however it isn't as significant as the contribution of the lipophilic components. The total extract HP4 should have an activity which averages the activity of the two fractions, and therefore it should show an LC₅₀ value in the middle of the corresponding values of the fractions. This indeed is the case as it is shown in Figure 5. Extract HP3, and fraction HP4 contain a significant amount of hypericins (1,32% and 1,37%). On the other hand in fraction HP5 hypericins concentration couldn't be detected, which means that either the concentration is below the detection limits, or there are no hypericins in the sample. This variation in the hypericins concentration in relation with the anti-tumor activity of the three samples HP3, 4, 5 indicate that hypericins are not contributing significantly in the anti-tumor activities of hypericum perforatum L. As it has already been stated hypericin is a novel photosensitizer used for the destruction of cancer in combination with light at the proper wavelength (31). The term hypericins is attributed to the total quantity of the naphtodianthrones found in the herb, since they absorb at 590 nm.

### Acknowledgements

This work was supported in part by a E.C. grant from General Secretary of Research & Technology of Greece (grant 35-Spin off 2001).

### References

1. Landis SH, Murray T, Bolden S and Wingo P. A: Cancer Statistics, 1998. CA Cancer J Clin 48: 6-29, 1998.
2. Spruck CH, Ohneseit PF and Gonzale-Zulueta M, Esrig D, Miyao N, Tsai YC, Lerner SP. Schmutte C, Yang AS and Cote R: Two molecular pathways to transitional cell carcinoma of the bladder, Cancer Res 54: 784-788, 1994.
3. Koss LG: Tumors of bladder. In: Diagnostic Cytology of the Urinary Tract With Histopathology and Clinical Correlations; Koss LG Ed: New York, Lippincott-Raven, pp 71-80, 1995.
4. Morgan DT, Bowsher W, Griffiths DF and Matthews PN: Rationalisation of follow-up in patients with non-invasive bladder tumors. A preliminary report. Br J Urol 67: 158-161, 1991.
5. Heney NM, Ahmed S, Flanagan MJ, Flanagan MJ, Frable W, Corder MP, Hafermann MD and Hawkins IR: National bladder collaborative group superficial bladder cancer: progression and recurrence. J Urol 130: 1083-1086, 1983.
6. Kurth KH, Boufoux C, Sylvester R, van der Meijden APM, Oosterlinck W, Brausi M: Treatment of superficial bladder tumors: achievements and needs. Eur Urol: 37 (suppl. 3): 1-9, 2000.
7. Prout GR Jr, Barton BA, Grifin PP and Friedell GH: Treated history of non-invasive grade 1 transitional cell carcinoma. J Urol 148: 1413-1419, 1992.
8. Olaiwola A: WHO Guidelines for the Assessment of Herbal Medicines. ESCOP Symposium: The scientific assessment of phytomedicines. Milan, Italy, 13 March, 1992. Fitoterapia vol 1, XIII, No. 2, 1992.
9. Baladrin MF, Kjocke JA, Wurtele ES and Bollinger WH: Natural plant chemicals: sources of industrial and medicinal materials. Science 228: 1154-1160, 1985.
10. O'Neil MJ and Lewis JA: In: Human Medicinal Agents from Plants; Kinghorn AD and Baladrin MF, Eds: ACS Symposium Series 534, American Chemical Society: Washington, D.C, 1993, pp 48-55.
11. Cragg GM, Newman DJ and Sander KM: Natural products in drug discovery and development. J Nat Prod 60: 52-60, 1997.
12. Cragg GM, Schepartz SA, Suffness M and Grever MR: The taxol supply crisis: new NCl policies for handling the large scale production of novel product anticancer and anti- HIV agents. J Nat Prod 56: 1657-1668, 1993.
13. The complete German Commission E monographs: therapeutic guide to herbal medicines. Blumenthal M, Busse WR, Goldberg A, Gruenwald J, Hall T, Riggins CW and Rister RS Eds: American Botanical Council, Boston, 1998.
14. Encyclopedia of common natural ingredients used in food, drugs and cosmetics. Leung, A Y and Foster S. Eds: John Wiley and Sons, New York, 1996.
15. St. John's Wort (Hypericum perforatum) In: American herbal pharmacopoeia (AHP); Upton R Ed: pp 1-32,1997.
16. Malamas M and Marselos M: The tradition of medicinal plants in Zagori, Epirus (Northwestern Greece). J of Ethnopharmacology 37: 197-203, 1992.
17. Brantner A, Della Loggia R, Sosa S and Kartnig T: Untersuchungen zur antiphlogistichen Wirkung von Hypericum perforatum L. Sci Pharm 62: 7-8, 1994.
18. Shakirova KK, Garagulya AD and Khazanovich RL: Antimicrobial properties of some species of St. John's wort cultivated in Uzbekistan. Mikrobiol Zh 32: 494-497, 1970.
19. Yip, L. Hudson JB, Gruszecka-Kowalik E, Zalkow IH and Neil towers GH: Antiviral activity of a derivative of the photosensitive compound hypericin. Phytomedicine III2: 185-190, 1996.
20. Harrer G and Sommer H: Treatment of mild/moderate depressions with Hypericum. Phytomedicine 1: 3-8, 1994.
21. Gaster B and Holroyd J: St John's wort for depression: a systematic review. Arch Intern Med 160: 152-156, 2000.
22. Greeson JM, Sanford B and Monti DA: St John's wort (Hypericum perforatum): a review of the current pharmacological, toxicological, and clinical literature. Psychopharmacology 153: 402-14, 2001.
23. National Center for Complementary and Alternative Medicine: St. John's wort fact sheet, publication Z-02. National Institute of Health, Bethesda, 1999.
24. Nahrstedt A and Butterweck V: Biologically active and other chemical constituents of the herb of Hypericum perforatum L. Pharmacopsychiatry 30S: 129-134, 1997.
25. Bilia AR, Gallori S and Vincieri FF: St John's wort and depression efficacy, safety and tolerability - an update. Life sciences 70: 3077-3096, 2002.
26. European Pharmacopoeia, Hypericum monograph, Supplement: 813-814, 2000.
27. United States Pharmacopoeia, St. John's wort monograph, 9^{th} Supplement: 4709-4710, 1998.
28. Singer A, Wonneman M and Mueller WE: Hyperforin, a major antidepressant constituent of St. John's wort inhibits serotonin uptake by elevating free intracellular Na+1, J. of Pharmacol. And Exp. Therapeutics 290: 1363-1368, 1999.
29. Butterweck V, Petereit F, Winterhoff H and Nahrstedt A: Solubilized-hypericin and pseudohypericin from hypericum perforatum exert anti-depressant activity in the forced swimming test. Planta Med 64: 291-294, 1998.
30. Butterweck V, Jurgenliemk G, Nahrsted A and Winterhoff H: Flavonoids from hypericum perforatum show antidepressant activity in the forced swimming test. Planta Med 66: 3-6, 2000.
31. Agostinis P, Vantieghem A, Merlevede W and de Witte AM: Review hypericin in cancer treatment: more light on the way. Int. J. of Biochemistry & Cell Biology 34: 221-241, 2002.
32. Koishi M, Hosokawa N, Sato M, Nakai A, Hirayoshi K, Hiraoka M, Abe M and Nagata K: Quercetin an inhibitor of head shock protein synthesis, inhibits the acquisition of thermotolerance in a human colon carcinoma cell line. Jpn J Cancer Res 83:1216-1222, 1992.
33. Scambia G, Ranelletti FO, Panici PB, Piantelli M, De Vincenzo R, Ferrandina G, Bonanno G, Capelli A and Mancuso S: Quercetin induces type-II estrogen-binding sites in estrogen-receptor negative (MDA-MB231) and estrogen-receptor-positive (MCF-7) human breast-cancer cell lines. Int J Cancer 54: 462-6, 1993.
34. Wei YQ, Zhao X, Kariya Y, Fukata H, Teshigawara K and Uchida A: Induction of apoptosis by quercetin: involvement of heat shock protein. Cancer Res 54: 4952-4957, 1994.
35. Hostanska K, Reichling J, Bommer S, Weber M and Saller R: Aqueous ethanolic extract of St. John's wort (Hypericum perforatum L.) induces growth inhibition and apoptosis in human malignant cells in vitro. Pharmazie 57: 323-331, 2002.
36. Mauri P and Pietta P: High performance liquid chromatography / electron spray mass spectrometry of hypericum perforatum extracts. Rapid Commun. Mass Spectrom. 14: 95-97, 2000.
37. Niedzwiedzki D and Gruszecki W.I.: Interaction between chlorophyll a and violaxanthin in different steric conformations: Model studies in monolayers. Colloids & Surfaces B: Biointerfaces 28: 27-38, 2003.
38. Schwaibold H, Pichlmeier U, Klingenberger HJ and Huland H: Long-term follow-up of cytostatic intravesical instillation in patients with superficial bladder carcinoma. Eur Urol 31: 153-159, 1997.
39. Kurth KH, Schroder FH, Tunn UAyR, Pavone-Macaluso M, Debruyne F, de Pauw M, Dalesio O and Kate F: Adjuvant chemotherapy for superficial transitional cell bladder carcinoma: long term results of a European Organization for research on treatment of cancer. Randomized trial comparing doxorubicin hydrochloride, ethoglucid and transurethral resection alone. J Urol 132: 258-62, 1984.
40. Lamm DL, Blumenstein BA, Crawford ED, Montie JE, Scardino P, Grossman HB, Stanisic TH, Smith JA, Sullivan J, Sarosdy MF: A randomized trial of intravesical doxorubicin and immunotherapy with bacillus Calmette-Guirin for transitional-cell carcinoma of the bladder. N Engl J Med, 325: 1205-1209, 1991.
41. Lundholm C, Norlen BJ, Ekman P, Jahnson S, Lagerkvist M, Lindeborg T, Olsson JL, Tveter K, Wijkstrom H, Westberg R, Malmstrom PU: A randomized prospective study comparing long-term intravesical instillations of mitomycin C and bacillus Calmette-Guirin in patients with superficial bladder carcinoma. J Urol, 156: 372-376, 1996.
42. Lamm DL, Steg A, Boccon-Gibod L, Morales A, Hanna MG Jr, Pagano F, Alfthan O, Brosman S, Fisher HA, Jakse G: Complications of bacillus Calmette-Guerin immunotherapy: review of 2602 patients and comparison of chemotherapy complications. Prog Clin Biol Res 310: 335-355, 1989.
43. Tei Y, Matsuyama H, Wada T, Kurisu H, Tahara M and Naito K: In vitro antitumor activity of bropirimine against urinary bladder tumor cells. Anticancer Res 22: 1667-1672, 2002.
44. Riggs DR, DeHaven JI and Lamn DL: Allium sativum (garlic) treatment for murine transitional cell carcinoma. Eur Urol 31 , Suppl 1: 27-30, 1997.
45. Ikemoto S, Sugimura K, Yoshida N, Yasumoto R, Wada S, Yamamoto K, and Kishimoto T: Antitumor effects of Scutellariae radix and its components baicalein, baicalin, and wogonin on bladder cancer cell lines. Urology 55: 951-955, 2000.
46. Su SJ, Yeh TM, Lei HY and Chow NH: The potential of soybean foods as a chemoprevention approach for human urinary tract cancer. Clin Cancer Res 6: 230-236, 2000.
47. Cragg GM, Boyd MR, Cardellina JH 2^{nd}, Newman DJ, Snader KM and McCloud TG: Ethnobotany and drug discovery: the experience of the UD National Cancer Institute. Ciba Found Symp 185: 178-190, 1994.
48. Von Eggelkraut-Gottanka SG, Abu Abed S, Muller W and Schmidt PC: Quantitative analysis of the active components and the by-products of eight dry extracts of hypericum perforatum L (st John's wort). Phytochem Anal 13: 170-176, 2002.
49. Dashwood R, Negishi T, Hayatsu H, Breinholt V, Hendricks J and Bailey G: Chemopreventive properties of chlorophylls towards aflatoxin B1: a review of the antitutagenicity and anticarcinogenicity data in rainbow trout. Mutation research/fundamental and molecular mechanisms of mutagenesis 399: 245-253, 1998.
50. Cervo L, Rozio M, Ekalle-Soppo CB, Guiso G, Morazzoni P and Caccia S: Role of hyperforin in the antidepressant-like activity of hypericum perforatum extracts. Psychopharmacology 164: 423-428, 2002.

## Claims

1. A lipophilic extract of Hypericum perforatum for use as a medicament to prevent and treat cancer.

2. A method of preventing and treating cancer comprising administering a pharmaceutically effective amount of a lipophilic extract of Hypericum perforatum.

3. A method of producing a pharmaceutically active lipophilic extract of Hypericum perforatum comprising:
(i) Extracting at least parts of Hypericum perforatum with a non-polar solvent; and
(ii) Removing the non-polar solvent from the lipophilic extract.

4. A method of producing a pharmaceutically active lipophilic extract of Hypericum perforatum according to claim 3 comprising:
(a) Forming an aqueous or alcoholic infusion of at least parts of Hypericum perforatum in an aqueous and/or alcoholic solvent; and
(b) Extracting the infusion with a non-polar solvent which is substantially immiscible with the aqueous and/or alcoholic solvent;
(c) Separating the non-polar solvent containing the lipophilic extract from the aqueous and/or alcoholic solvent; and
(d) Removing the non-polar solvent from the lipophilic extract.

5. A method according to claim 3 or claim 4, wherein the alcoholic solvent is methanol.

6. A method according to claims 3 to 5, wherein the non-polar solvent is petroleum ether.

7. A method according to any one of claims 3 to 6, additionally comprising the step further purifying the lipophilic extract to identify one or more compounds from that extract having anticancer activity.

8. A method according to any one of claims 3 to 7, additionally comprising the step of mixing the lipophilic extract with one or more pharmaceutically acceptable carriers, adjuvants or vehicles.

9. A pharmaceutically active composition obtainable by a method according to any one of claims 3 to 8.

10. A pharmaceutically active composition according to claim 9 for use as a medicament to treat cancer.

11. A method of treating cancer comprising administering a pharmaceutically effective amount of an extract according to claim 8.

12. Use of a method according to claims 1, 2, 10 or 11, wherein the cancer is a bladder cancer.

13. A method according to claim 12, wherein the extract is administered into the bladder

## Amended claims

### Amended claims in accordance with Rule 86(2) EPC.

**1.** Use of a lipophilic extract of Hypericum perforatum for the preparation of a medicament containing a pharmaceutically effective amount to prevent and treat bladder cancer.

**2.** A method of producing the pharmaceutically active lipophilic extract of Hypericum perforatum according to claim 2 comprising:
(a) Forming an aqueous or alcoholic infusion of at least parts of Hypericum perforatum in an aqueous and/or alcoholic solvent; and
(b) Extracting the infusion with a non-polar solvent which is substantially immiscible with the aqueous and/or alcoholic solvent;
(c) Separating the non-polar solvent containing the lipophilic extract from the aqueous and/or alcoholic solvent; and
(d) Removing the non-polar solvent from the lipophilic extract.

**3.** A method according to claim 2, wherein the alcoholic solvent is methanol.

**4.** A method according to claims 2 to 3, wherein the non-polar solvent is petroleum ether.

**5.** A method according to any one of claims 2 to 4, additionally comprising the step further purifying the lipophilic extract to identify more active fraction of the extract.

**6.** A method according to any one of claims 2 to 6, additionally comprising the step of mixing the lipophilic extract produced in claims 2 to 5 with one or more pharmaceutically acceptable carriers, adjuvants or vehicles.

**7.** Use of a pharmaceutically active composition obtainable by a method according to any one of claims 2 to 7 to prevent and treat bladder cancer, wherein the extract is administered into the bladder.
